# EUROPÄISCHE PATENTANMELDUNG

(11) **EP 4 685 129 A1**
(43) Veröffentlichungstag der Anmeldung: **28.01.2026**
(21) Anmeldenummer: 24191090.0
(22) Anmeldetag: 26.07.2024
(51) Int. Cl.: C07C 209/68, C07C 211/36

(54) **VERFAHREN ZUR HYDRIERUNG VON MDA**

(71) Anmelder: Evonik Operations GmbH, 45128 Essen (DE)
(72) Erfinder: BOECK, Florian, 58455 Witten (DE); SCHNIEDER, Sven, 45711 Datteln (DE); LUDWIG, Martina, 45657 Recklinghausen (DE)
(74) Vertreter: Evonik Patent Association

(57) **Zusammenfassung**

Gegenstand der vorliegenden Erfindung ist ein Verfahren zur kontinuierlichen, heterogenen katalytischen Hydrierung von MDA, bei dem
a) das Verfahren in einer Reaktorkaskade durchgeführt wird, die n seriell verschaltete, jeweils mit Katalysator befüllte, unabhängig voneinander befüll- und entleerbare Reaktionsräume aufweist,
die in der Reihenfolge ihrer Verschaltung jeweils als *Rᵢ* mit 1 ≤ i ≤ n bezeichnet werden,
b) und R₁ zeitweilig aus der Verschaltung herausgenommen wird, sobald der in R₁ befindliche Katalysator im Verlauf der Reaktion zu einem unerwünschten Ausmaß deaktiviert wurde,
∘ so dass eine neu verschaltete Reaktorkaskade erhalten wird, die i' Reaktionsräume aufweist, die in der Reihenfolge ihrer Verschaltung jeweils als *R_{i'}* mit 1 ≤ i' ≤ (n - 1) bezeichnet werden,
∘ wobei jeder Reaktionsraum Rᵢ mit 2 ≤ i ≤ n zu einem Reaktionsraum R_{i'} mit 1 ≤ i' ≤ (n - 1) wird,

c) der Katalysator in R₁ getauscht und/oder regeneriert und
d) nachfolgend R₁ als Reaktionsraum R_{i'} mit i' = n verschaltet wird.

## Beschreibung

Die vorliegende Erfindung betrifft ein Verfahren zur Hydrierung von MDA.

Methylenbis(cyclohexylamin) ist eine bedeutende Industriechemikalie, die bei vielen typischen Aminreaktionen eingesetzt wird, wie Reaktionen mit Carboxylsäuren, Phosgen, Aldehyden, Ketonen und Epoxiden. Mit Methylenbis(cyclohexylamin) können die Vorteile cycloaliphatischer Amine in Epoxysystemen Anwendung finden: niedrige Mischviskositäten, moderate Reaktivität und wenig exothermes Verhalten sowie herausragende mechanische Eigenschaften und exzellente Chemikalienbeständigkeit. Im Vergleich zu anderen Aminen ist die Neigung zur Carbamatbildung vermindert, was einen Vorteil für die Verwendung als Epoxidhärter darstellt.

Methylenbis(cyclohexylamin) ist ein bei Standardbedingungen (SATP) festes oder flüssig vorliegendes cycloaliphatisches Amin, das üblicherweise über die Flüssigphasen-Hydrierung von MDA hergestellt wird. Das Akronym MDA wurde historisch als Abkürzung für das bei der Umsetzung von Anilin und Formaldehyd gebildete, vor allem "Methylendianilin" (Diaminodiphenylmethan) umfassende Produktgemisch eingeführt und wird weiterhin zur Bezeichnung des mittlerweile großtechnisch erzeugten Verfahrensproduktes verwendet. Das Produkt der Hydrierung, das vor allem Methylenbis(cyclohexylamin) umfasst, wird deswegen oft auch als H12MDA bezeichnet.

MDA ist aufgrund seines Herstellungsprozesses üblicherweise ein Gemisch aus verschiedenen Diaminodiphenylmethanen. Vor allem besteht es aus 4,4`-Diaminodiphenylmethan. Es können jedoch auch 2,4`- und 2,2'-lsomere vorliegen. MDA kann weiterhin bei der Umsetzung von Anilin und Formaldehyd gebildete Reaktionsprodukte mit drei oder mehr aromatischen Ringen, insbesondere solche mit drei oder mehr Phenylringen, enthalten. Diese Reaktionsprodukte mit drei oder mehr aromatischen Ringen werden auch als Mehrkernverbindungen bezeichnet.

Bei dem im Handel erhältlichen Methylenbis(cyclohexylamin) handelt es sich aufgrund des hohen Anteils an 4,4`-Diaminodiphenylmethan im eingesetzten MDA größtenteils um 4,4`-Diaminodicyclohexylmethan bzw. Bis(para-Aminocyclohexyl)methan. Aufgrund der potentiellen Anwesenheit der entsprechenden 2,4`- und 2,2'-Diaminophenylmethan-lsomere im MDA kann in Methylenbis(cyclohexylamin) auch 2,4'-Diaminodicyclohexylmethan und 2,2`-Diaminodicyclohexylmethan vorliegen. Weiterhin kann hydriertes MDA neben Methylenbis(cyclohexylamin) weiterhin noch (ggf. partiell) hydrierte Mehrkernverbindungen enthalten.

US 5,578,546 A offenbart, dass 1947 erstmalig ein Verfahren zur Herstellung von Methylen- bis(cyclohexylamin) beschrieben und 1965 in den technischen Maßstab überführt wurde.

Die Hydrierung von MDA ist stark exotherm. So gibt WO 2010/069484 A1 eine Reaktionsenthalpie von - 1600 kJ/mol an.

In Folge der Hydrierung werden je nach Verfahren verschiedene Diastereoisomere gebildet. Dabei kann das von 4,4`-Diaminodiphenylmethan abgeleitete Produkt 4,4'-Diaminodicyclohexylmethan als trans/trans-, cis/cis- und cis/trans-Isomer vorliegen und ist deswegen in der Regel ein Gemisch dieser Isomere mit unterschiedlichen Anteilen. Mit steigendem trans/trans-Gehalt steigt dabei der Schmelzpunkt der Verbindung. Deshalb unterscheiden sich die Einsatzgebiete je nach Isomerengehalt stark: Während Methylenbis(cyclohexylamin)-Qualitäten mit niedrigem trans/trans -Gehalt (z. B. 10 - 30 Gew.-%) im Bereich der Amin- und Isocyanat-Vernetzer, insbesondere im Bereich von Zwei-Komponenten-Harzen Einsatz finden, finden Qualitäten mit hohem trans/trans -Gehalt (z. B. ≥ 48 Gew.-%) vor allem Anwendung als Regulator in Polyamidverbindungen. Gerade die Herstellung von Produkten mit niedrigem trans/trans-Gehalt stellt eine Herausforderung dar, da das thermodynamische Gleichgewicht, wie in US 3,636,108 A beschrieben wird, im Bereich wesentlich höherer trans/trans-Anteile (bis zu 51,2 %) liegt. US 2,606,925 A zeigt zudem, dass das Gleichgewicht nachträglich durch längeres Temperieren in Richtung eines höheren Anteils an trans/trans-Isomer verschoben werden kann.

Die Zusammensetzung des Hydrierungsproduktes hängt auch von der Zusammensetzung des eingesetzten MDAs ab: Oft wird MDA in Qualitäten von MDA50 bis MDA100 eingesetzt, wobei die zwischen 50 und 100 liegende Zahl den Gehalt an Diaminodiphenylmethanen im MDA-Gemisch angibt. MDA50 ist dabei eine MDA-Qualität, die wie oben erläutert prozessbedingt etwa 50 Gew.-% Diaminodiphenylmethanen und 50 Gew.-% Mehrkernverbindungen enthält. Die einzelnen Mehrkernverbindungen können entsprechend der Zahl enthaltener aromatischer Ringe als 3-Kern-Verbindungen, 4-Kern-Verbindungen, etc. bezeichnet werden. MDA50 ist dabei die am meisten produzierte Qualität und wird hauptsächlich zu Methylendicyclohexyldiisocyanat (MDI) weiterverarbeitet. MDA100 ist reines MDA bzw. Diaminodiphenylmethan ohne Mehrkernverbindungen. MDA85 oder MDA90 sind andere auf dem Markt verfügbare Qualitäten mittlerer Reinheit. Wenn in Patentschriften zum Herstellverfahren von Methylenbis(cyclohexylamin) auf die Reinheit der MDA-Qualität eingegangen wird, so ist meist von MDA100 die Rede (z. B. CN 110204447 B). Dagegen stellt US 2005/261525 A1 bevorzugt auf die Hydrierung von MDA50 ab. Die dabei als Hochsieder anfallenden hydrierten, oligomeren Amine eignen sich als Vernetzer mit besonders niedrigen Dampfdrücken für eine Reihe von Spezialanwendungen, wie US 2004/162409 A1 zeigt.

Der Gehalt an (ggf. partiell) hydrierten Mehrkernverbindungen im Produkt nimmt in der Reihenfolge der Edukte MDA50, MDA85, MDA90, MDA100 ab, da der Gehalt an Mehrkernverbindungen von MDA50 zu MDA100 abnimmt.

In der Literatur beschrieben sind Hydrierungen von MDA mit Katalysatoren mit aktiven Metallen ausgewählt aus Cobalt (z. B. US 3,743,677 A), Nickel (z. B. US 4,503,251 A), Ruthenium (z. B. US 2,494,563 A, US 2,606,925 A, US 2,606,928 A, US 3,959,374 A, US 3,636,108 A und US 4,161,492 A), Rhodium (z. B. DE 24 23 639 C3) und Iridium (z. B. US 3,914,307 A) oder Kombinationen aus diesen.

Für die Erzielung von Produkt mit niedrigem trans/trans-Gehalt bei hohen Umsätzen und guten Selektivitäten haben sich vor allem Katalysatoren auf Ruthenium- und Rhodium-Basis etabliert. Diese Katalysatoren werden in neueren Patentschriften vor allem als geträgerte Katalysatoren eingesetzt. So offenbart FR 2372142 A1 einen Katalysator auf Aluminiumoxid, wohingegen in US 2016/304436 A1 Zirconiumoxid als Träger eingesetzt wird. Es wird vielfach beschrieben, dass der Träger, der BET-Wert des Trägers und die Porengröße einen entscheidenden Einfluss auf die Lebenszeit des Katalysators haben (z. B. US 5,773,657 A, CN 102008969 B, US 2002/087036 A1, US 2004/034252 A1). Teilweise werden bewusst basische Träger verwendet. So beschreibt US 5,578,546 A1 einen Katalysator auf basischer Tonerde, wohingegen US 6,184,416 B1 Lithiumaluminiumoxid als basischen Träger offenbart.

In vielen der bereits genannten Literaturstellen wird die geringe Lebenszeit des Katalysators als die größte Schwachstelle des Verfahrens bezeichnet. US 3,636,108 A berichtet von einer stetig sinkenden Aktivität des Katalysators, die auf Verkokung oder Verblockung der aktiven Oberfläche mit oligomeren Verbindungen zurückgeführt wird. Es wird offenbart, dass dieser Deaktivierungsprozess durch Zugabe von Ammoniak und Alkalimetall reduziert werden kann. Ammoniak in Verbindung mit einem aliphatischen Alkohol als Additiv wird auch von US 5,214,212 A beschrieben.

Die Verwendung von Alkali- und/oder Erdalkalimetall als Promotoren zur Steigerung von Aktivität, Selektivität und/oder Lebenszeit wird auch von anderen Patentschriften aufgezeigt: US 3,697,449 A offenbart den positiven Effekt in situ als Promotor zugesetzter Alkalihydroxide oder -alkoxide auf die Hydrierung von MDA und anderen aromatischen Aminen. JP 2002/348267 A1 offenbart die Zugabe von Ca(OH)₂ als basischen Moderator zur Vermeidung von aufwendigen Regenerierungsverfahren für den Katalysator. US 6,184,416 B1 zeigt die höhere Aktivität eines Rhodium-Katalysators, der direkt auf einen Lithiumaluminiumoxidträger aufgebracht wurde. In JP H08-092175 A wird ein System beschrieben, bei dem dem Edelmetallkatalysator Alkalicarbonat in Diethylenglycoldimethylether als Lösungsmittel zugegeben wird. US 6,075,167 A zeigt, dass durch Zugabe von Metallnitrit die Reaktionsgeschwindigkeit verkürzt und die Bildung von Nebenprodukten reduziert werden kann. US 4,448,995 A setzt Nitrate oder Sulfate von Alkali- oder Erdalkalimetallen als Moderatoren ein. US 4,186,145 A offenbart die Kombination von organischen und/oder organischen Alkalimetallverbindungen zusammen mit Oxiden, hydratisierten Oxiden oder Hydroxyden von Chrom und Mangan als Promotoren. CN 111804324 B offenbart, dass durch Zugabe von Lithiumamid die Oligomerisierung von Methylenbis(cyclohexylamin) und die Aminoalkoholbildung reduziert werden kann. Als Vorteil gegenüber anderen Lithiumsalzen wird angegeben, dass dieses besser löslich in einem organischen Lösungsmittel sei. CN 116023272 A beschreibt letztlich den Einsatz von Lithiumformiat, -acetat oder oxalat als Promotor zur Erhöhung des Durchsatzes und zur Steuerung des trans/trans-Gehalts in einer Rührkesselkaskade.

All die beschriebenen Zugaben von Alkalisalzen haben den Nachteil, dass sich diese im weiteren Prozess in den Hochsiedern anreichern, welche dadurch nicht mehr als Wertprodukt verwendet werden können. Zudem haben die beschriebenen Additive den Nachteil, korrodierend auf den Reaktor zu wirken.

Auch organische Additive zur Erhöhung der Lebenszeit wurden wiederholt beschrieben (z. B. CN 106631826 B, CN 103265438 B oder CN 115870013 A). Nachteilig ist auch hier, dass diese Produkte als Verunreinigungen im Prozess verbleiben.

Eine weitere Variante zur Erhöhung der Lebenszeit offenbart US 2010/292510 A1. Hier werden anorganische Additive wie zum Beispiel Aluminiumoxid oder Siliziumoxid zur Reaktion gegeben, um Katalysatorgifte zu binden. In US 6,998,507 B1 wird MDA in Gegenwart von Wasserstoff in Gegenwart eines Rutheniumkatalysators partiell hydriert und dann in Gegenwart eines Rhodiumkatalysators vollständig umgesetzt. Dabei wird der Rutheniumkatalysator ebenfalls als Adsorbens für Katalysatorgifte verwendet. Die zweistufige Reaktionsführung verkompliziert jedoch den Prozess.

Generell ist auch mit Promotoren oder Moderatoren die Lebenszeit der eingesetzten Katalysatoren im Vergleich zu anderen Prozessen stark limitiert. Eingebrachte Oligomere aus dem MDA selbst oder aus dem Hydrierungsprozess belegen nach und nach die Oberfläche von Katalysatoren und inhibieren diese. In Suspensionsprozessen wird zur Aufrechterhaltung häufig über die Zeit Katalysator nachdosiert. Dieses ist für kontinuierliche Festbettverfahren nicht möglich. Mehrere Patentschriften beschreiben daher spezielle Regenerationsverfahren für die eingesetzten Katalysatoren.

So beschreibt CN 117654548 A die Regenerierung des Katalysators zur Herstellung von Methylenbis(cyclohexylamin) aus MDA durch Heißbehandlung mit langkettigem Alkohol, anschließender Wäsche mit einem polaren Lösungsmittel und Aktivierung im Wasserstoffstrom.

CN 110204447 B beschreibt ein Regenerationsverfahren für einen zur MDA-Hydrierung eingesetzten Katalysator, bei dem der Katalysator unter anderem mehrfach mit alkalimetallhaltigem Ammoniak gewaschen und anschließend hoch erhitzt wird.

In CN 113893866 B wird offenbart, dass die Aktivität des Katalysators durch Waschen mit Säure und Wasser in inerter Atmosphäre und anschließender Reaktivierung zurückerhalten werden kann.

Regenerationsverfahren sind jedoch aufgrund des damit verbundenen Aufwandes nachteilig.

Verfahrenstechnisch wurden sowohl Hydrierungen vom MDA mit suspendierten Katalysatoren als auch solche im Festbettverfahren beschrieben. Beide Verfahren haben ihre Vor- und Nachteile.

So ist in einem Rührkessel die Nutzungsrate des suspendierten Katalysators sehr hoch, allerdings ist das Produkt in diesem Fall auch sehr lange hohen Temperaturen ausgesetzt und neigt zu einer Isomerisierung zu trans/trans-angereichertem Produkt. Zudem sind Rührkesselreaktoren in Bezug auf den Massentransport von Gasphase zum festen Katalysator limitiert. Außerdem beinhalten Rührkesselreaktionen die Gefahr, dass Reaktionswärme zum einen nur schlecht abgeführt werden kann und zum anderen die Reaktion, einmal gestartet, nicht abgebrochen werden kann.

CN 117623940 A beschreibt, dass ein Methylenbis(cyclohexylamin)-Produkt mit niedrigem trans/trans-Isomerengehalt bei hohem Umsatz auch bei langer Verwendung des suspendierten Katalysators in einer Rührkesselkaskade erreicht werden kann, wenn bei sinkendem Umsatz die Feedmenge reduziert wird.

EP 231 788 B1 beschreibt ein Verfahren zur Hydrierung von MDA in Gegenwart eines Rhodium- und eines Ruthenium-haltigen Suspensions-Katalysators. Es wird weiterhin offenbart, dass bei einem vergleichsweise niedrigem Wasserstoffdruck von etwa 50 bar ein Produkt mit besonders niedrigem trans/trans-Gehalt bei hohen Umsätzen erzielt werden kann. Gleichzeitig werden sehr hohe Lebenszeiten und eine geringe Neigung zur Oligomerisierung erreicht. Ein Nachteil des Verfahrens ist, dass der Einsatz von Rhodium sehr hohe Temperaturen erfordert.

Generell nachteilig an Suspensions-Katalysatoren ist, dass ihre Abtrennung, insbesondere bei kontinuierlichen Verfahren, technisch aufwändig ist.

So beschreibt EP 1 251 119 A2 die Herstellung von Diaminodicyclohexylmethan mit einem mit niedrigem trans/trans-Gehalt in einem kontinuierlich betriebenen Suspensionsreaktor. Bevorzugt wird die Reaktion in einer Rührkesselkaskade durchgeführt. Nachteilig daran ist, dass die Hydrierung nur mit einem sehr großen technischen Aufwand so durchgeführt werden kann, dass MDA vollständig hydriert wird.

EP 2 285 481 A1 offenbart eine Kombination aus Schlaufenreaktor und Blasensäule für eine möglichst kontrollierte Reaktion. Mit Hilfe des Schlaufenreaktors soll die Reaktionswärme effektiv aus dem Prozess zu entfernen sein. Allerdings ist ein Schlaufenreaktor insbesondere limitiert in Bezug auf den Massentransport.

Zur Erzielung vorteilhafter Eigenschaften kann MDA auch in Festbettreaktoren hydriert werden. Insbesondere als Rieselbettreaktor ausgestaltete Festbettreaktoren haben den Vorteil, dass sie einen sehr guten Massentransport ermöglichen und dadurch weniger Nebenprodukte generieren. Zusätzlich verbleibt das umgesetzte Produkt möglichst kurz in Kontakt mit dem Katalysator, wodurch ebenfalls Nebenreaktionen - in diesem Fall die Isomerisierung - reduziert werden. Rohrbündelreaktoren haben weiterhin den Vorteil, dass bei ihnen das Reaktionsmedium ausgesprochen effizient gekühlt werden kann. Nachteilig an kontinuierlich betriebenen Festbettreaktoren ist jedoch, dass die Produktion unterbrochen werden muss, wenn die Aktivität des Katalysators nachlässt und er ausgetauscht bzw. aufbereitet werden muss.

CN 116621711 B beschreibt ein kontinuierliches Verfahren zur Herstellung von 4,4`-Diaminodicyclohexylmethan, bei dem die Hydrierung in einem Festbettreaktor durchgeführt wird und die Reaktion bei einem Restgehalt von 0 - 10 % MDA abgebrochen und Produkt destillativ aufgereinigt wird. Die nicht vollständig hydrierten Komponenten werden entweder nachhydriert oder in den Prozess zurückgeführt. Dies ermöglicht, dass trotz hoher Temperaturbelastung der gewünscht niedrige trans/trans-Methylenbis(cyclohexylamin)-Anteil erhalten wird. Nachteilig an diesem Verfahren ist, dass die Produktion unterbrochen werden muss, wenn die Aktivität des Katalysators nachlässt und er ausgetauscht bzw. aufbereitet werden muss.

WO 2010/069484 A1 offenbart ein Verfahren zur Herstellung von Bis(para-Aminocyclohexyl)Methan aus Methylendianilin, bei dem die Reaktion in 5 bis 50 nacheinander geschalteten Reaktionszonen, in denen die heterogenen Katalysatoren vorliegen, unter adiabatischen Bedingungen ausgeführt wird. Bevorzugt werden Festbettkatalysatoren eingesetzt. Experimentelle Daten werden hierzu nicht bereitgestellt. Die hohe Anzahl an verschalteten Reaktoren und die adiabatische Reaktionsführung sind jedoch für die großtechnische praktische Anwendung zu aufwändig und machen dieses Verfahren wirtschaftlich wenig attraktiv. Weiterhin ist auch an diesem Verfahren nachteilig, dass die Produktion unterbrochen werden muss, wenn die Aktivität des Katalysators nachlässt und er ausgetauscht bzw. aufbereitet werden muss.

In WO 2009/144148 A1 wird unter anderem die Hydrierung aromatischer Diamine zu cycloaliphatischen Diaminen und insbesondere die Hydrierung von MDA zu Methylendicyclohexyldiamin offenbart. Eine bevorzugte Ausführungsform offenbart die Hydrierung aromatischer Amine und insbesondere von MDA in Festbettreaktoren. Es wird offenbart, dass es sich als vorteilhaft herausgestellt hat, die Reaktion in zwei oder mehr in Serie geschalteten Reaktionsräumen durchzuführen, da diese unabhängig voneinander temperierbar seien und auch ein partieller Katalysatorwechsel möglich sei. Auch an diesem Verfahren ist nachteilig, dass die Produktion unterbrochen werden muss, wenn die Aktivität des Katalysators nachlässt und er ausgetauscht bzw. aufbereitet werden muss.

WO 2015/086638 A1 offenbart ein Verfahren zur Hydrierung von 4,4'-Methylendianilin und/oder Polymer-MDA mit Wasserstoff in Gegenwart eines Zirkoniumoxid-geträgerten Rutheniumkatalysators. Die Umsetzung kann kontinuierlich und im Festbett durchgeführt werden. Bevorzugt führt man das Verfahren in Rieselreaktoren oder in gefluteter Fahrweise nach der Festbettfahrweise durch. Um einen vollständigen Umsatz zu erzielen, kann eine Nachreaktion des Hydrieraustrags erfolgen. Dazu kann der Hydrieraustrag durch einen oder mehrere nachgeschaltete Reaktoren geleitet werden, die mit dem erfindungsgemäßen oder einem anderen Katalysator befüllt sind. Auch an diesem Verfahren ist nachteilig, dass die Produktion unterbrochen werden muss, wenn die Aktivität des Katalysators nachlässt und er ausgetauscht bzw. aufbereitet werden muss.

Den im Stand der Technik bekannten Verfahren ist gemein, dass sie zu aufwändig sind, schlechte Ausbeuten liefern und/oder aufgrund der Verwendung von Zusatzstoffen zu Produkten mit negativen Eigenschaften führen und/oder zu einer Unterbrechung führen, weil von Zeit zu Zeit Katalysator aufbereitet werden muss. Dies Problem ist besteht insbesondere bei der Verwendung von Festbettkatalysatoren.

Es ist somit die Aufgabe der vorliegenden Erfindung, die bestehenden Nachteile zu überwinden. Insbesondere ist die Aufgabe der vorliegenden Erfindung, ein Verfahren zur kontinuierlichen Herstellung von Methylenbis(cyclohexylamin) zu entwickeln, das ein hohes Maß an Prozesssicherheit mit einer höheren spezifischen Standzeit des Katalysators verbindet. Dabei soll auf die Zugabe von zusätzlichen betriebsfremden Stoffen verzichtet werden, die hochwertige Stoffströme im Hochsieder kontaminieren.

Die spezifischen Standzeit des Katalysators ist dabei die Menge an Methylenbis(cyclohexylamin) in kg, die pro kg Katalysator erzeugt werden kann, bevor dieser ausgetauscht werden muss.

Gelöst werden die sich vorliegend stellenden Aufgaben durch das erfindungsgemäße Verfahren zur kontinuierlichen, heterogenen katalytischen Hydrierung von MDA, bei dem
a) das Verfahren in einer Reaktorkaskade durchgeführt wird, die n seriell verschaltete, jeweils mit Katalysator befüllte, unabhängig voneinander befüll- und entleerbare Reaktionsräume aufweist, die in der Reihenfolge ihrer Verschaltung jeweils als *Rᵢ* mit 1 ≤ i ≤ n bezeichnet werden,
b) und R₁ zeitweilig aus der Verschaltung herausgenommen wird, sobald der in R₁ befindliche Katalysator im Verlauf der Reaktion zu einem unerwünschten Ausmaß deaktiviert wurde,
   ∘ so dass eine neu verschaltete Reaktorkaskade erhalten wird, die i' Reaktionsräume aufweist, die in der Reihenfolge ihrer Verschaltung jeweils als R;- mit 1 ≤ i' ≤ (n - 1) bezeichnet werden,
   ∘ wobei jeder Reaktionsraum Rᵢ mit 2 ≤ i ≤ n zu einem Reaktionsraum R_{i'} mit 1 ≤ i' ≤ (n - 1) wird,
c) der Katalysator in R₁ getauscht und/oder regeneriert und
d) nachfolgend R₁ (enthaltend den getauschten und/oder regenerierten Katalysator) als Reaktionsraum R_{i'} mit i' = n verschaltet wird.

Weiterhin hat das erfindungsgemäße Verfahren auch den Vorteil, dass die Menge an Katalysator, die ausgetauscht und/oder regeneriert wird, minimiert wird. Dies ist ressourcenschonend und vermindert weiterhin den Arbeitsaufwand. Zudem ermöglicht das erfindungsgemäße Verfahren den kontinuierlichen Betrieb der Anlage, da der Austausch des Katalysators in nur einem Reaktionsraum erfolgt und der Betrieb in den anderen Reaktionsräumen aufrecht erhalten bleibt, was die Abstellungszeit der Anlage deutlich reduziert.

### Verfahren zur Hydrierung von MDA

Das erfindungsgemäße Verfahren ist ein Verfahren zur Hydrierung von MDA. MDA, ein für "Methylendianilin" stehendes Akronym, bezeichnet eine Diaminodiphenylmethan-haltige Zusammensetzung. Diese stammt üblicherweise aus der Umsetzung von Anilin und Formaldehyd. Bevorzugt enthält die eingesetzte Diaminodiphenylmethan-haltige Zusammensetzung als Hauptbestandteil 4,4`-Diaminodiphenylmethan oder besteht daraus. Weiter bevorzugt enthält die eingesetzte Diaminodiphenylmethan-haltige Zusammensetzung 4,4`-Diaminodiphenylmethan als Hauptbestandteil sowie 2,4`-Diaminodiphenylmethan und 2,2`-Diaminodiphenylmethan als Nebenbestandteil. Neben 4,4`-Diaminodiphenylmethan, 2,4`-Diaminodiphenylmethan und 2,2`-Diaminodiphenylmethan können jedoch auch noch bei der Umsetzung von Anilin und Formaldehyd gebildete Reaktionsprodukte mit drei oder mehr aromatischen Ringen, insbesondere solche mit drei oder mehr Phenylringen ("Mehrkernverbindungen") vorliegen.

Bevorzugt wird bei dem vorliegenden Verfahren ein MDA eingesetzt, das mindestens 70 Gew.-% 4,4'-Diaminodiphenylmethan und 0,01 bis 2 Gew.-% N-Methylverbindungen (2,2'-, 2,4'- und/oder 4,4`-N-Methyl-methylendianilin, insbesondere 2,4'- und 4,4'-N-Methyl-Methylendianilin), jeweils bezogen auf die Gesamtmasse an Verbindungen mit aromatischen Ringen, aufweist. Noch weiter bevorzugt wird ein MDA bestehend aus 74 - 85 Gew.-% 4,4'-MDA, 3 - 20 Gew.-% 2,4'-MDA, weniger als 1 Gew.-% 2,2'-MDA und bis zu 1 Gew.-% N-Methylverbindungen eingesetzt. Mit den genannten Gemischen kann besonders gut ein Hydriergemisch erhalten werden, das einen trans/trans-Anteil des 4,4'-lsomeren von 10 bis 30 % aufweist.

Bei dem über die erfindungsgemäße Hydrierung erhältlichen Methylenbis(cyclohexylamin) handelt es sich aufgrund des hohen Anteils an 4,4`-Diaminodiphenylmethan im eingesetzten MDA größtenteils um 4,4'-Diaminodicyclohexylmethan bzw. Bis(para-Aminocyclohexyl)methan. Aufgrund der potentiellen Anwesenheit der entsprechenden 2,4`- und 2,2'-Diaminophenylmethan-Isomere im MDA kann in Methylenbis(cyclohexylamin) auch 2,4`-Diaminodicyclohexylmethan und 2,2`-Diaminodicyclohexylmethan vorliegen. Weiterhin kann hydriertes MDA neben Methylenbis(cyclohexylamin) weiterhin noch (ggf. partiell) hydrierte Mehrkernverbindungen enthalten.

In Folge der Hydrierung werden Diastereoisomere gebildet. Das von 4,4`-Diaminodiphenylmethan abgeleitete Produkt 4,4'-Diaminodicyclohexylmethan kann als trans/trans-, cis/cis- und cis/trans-Isomer vorliegen und ist deswegen in der Regel ein Gemisch dieser Isomere mit unterschiedlichen Anteilen. Besonders gut geeignet, vor allem im Falle des Einsatzes der o.g. MDA-Qualitäten, ist das erfindungsgemäße Verfahren zur Herstellung von 4,4'-Diaminodicyclohexylmethan-Gemischen mit hohen trans/trans-Anteilen.

Das erfindungsgemäße Verfahren zur Hydrierung von MDA ist ein weiter bevorzugt ein Festbettverfahren zur kontinuierlichen katalytischen Hydrierung. Weiter bevorzugt wird somit MDA mittels katalytischem Festbettverfahren in Gegenwart von Wasserstoff hydriert.

Bevorzugt wird pro Tonne Katalysator in der Reaktorkaskade ein Stoffstrom von 0,1 - 10 t/h, weiter bevorzugt 0,5 - 8 t/h, noch weiter bevorzugt 0,75 - 5 t/h eingeleitet. Der Stoffstrom besteht weiter bevorzugt aus einer Lösung enthaltend 5 - 50 Gew.-%, weiter bevorzugt 7,5 - 30 Gew.-%, noch weiter bevorzugt 10 - 20 Gew.-% MDA.

Der für die Hydrierung erforderliche Wasserstoff (H₂) wird bevorzugt stöchiometrisch bis minimal überstöchiometrisch in Bezug auf die gewünschte Reaktion zugegeben. Für das Verfahren zur Hydrierung von MDA wird der für die Hydrierung erforderliche Wasserstoff (H₂) in einem molaren Verhältnis von 300 - 350 mol-%, weiter bevorzugt 300 - 330 mol-%, weiter bevorzugt 300 - 310 mol-% bezogen auf die anwesenden Phenylringe im MDA zugegeben.

Die Hydrierung erfolgt bevorzugt bei Temperaturen zwischen 50 und 200 °C, vorzugsweise zwischen 80 und 170 °C, besonders bevorzugt zwischen 85 und 135 °C. Der Wasserstoffdruck liegt dabei bevorzugt zwischen 1 und 30 MPa, bevorzugt zwischen 5 und 15 MPa, besonders bevorzugt zwischen 7 und 10 MPa.

Bevorzugt handelt es sich bei dem erfindungsgemäßen Verfahren somit um ein Flüssigphasen-Hydrierverfahren, d. h. um ein Hydrierverfahren, das in der flüssigen Phase durchgeführt wird.

Prinzipiell kann bei der Hydrierung ein Lösemittel anwesend sein, es muss es aber nicht. Bevorzugt wird jedoch MDA in einem Lösungsmittel zugegeben. Der Anteil des Lösungsmittels liegt weiter bevorzugt zwischen 50 und 95 Gew.-%, noch weiter bevorzugt zwischen 70 und 92,5 Gew.-%, noch weiter bevorzugt zwischen 80 und 90 Gew.-% Lösemittel. Bevorzugte Lösungsmittel können ausgewählt werden aus der Gruppe bestehend aus primären, sekundären und tertiären ein- oder mehrwertigen Alkoholen (insbesondere Methanol, Ethanol, n- und i-Propanol, 1-, 2-, i- und tert.-Butanol, Ethylenglykol, und Ethylenglykolmono(C1-C3)alkylether), linearen Ethern (insbesondere Ethylenglykoldi(C1-C3)alkylether), cyclischen Ethern (insbesondere Tetrahydrofuran und Dioxan) und Alkanen (insbesondere n- und iso-Alkane mit 4-12 C-Atomen, weiter bevorzugt n-Pentan, n-Hexan und Isooctan, und cyklischen Alkanen, weiter bevorzugt Cyclohexan und Dekalin). Während Alkohole zu einer Alkylierung der Aminogruppen führen können, weisen Ether diesen Nachteil nicht auf und sind somit besonders bevorzugt. Ganz besonders bevorzugtes Lösungsmittel ist Tetrahydrofuran.

Lösungsmittel kann jedoch auch ebenfalls bevorzugt das Hydrierprodukt selbst sein.

Die Hydrierung kann bevorzugt auch in Gegenwart von Ammoniak, einem primären, sekundären oder tertiären Amin oder einem polycyklischen Amin mit einem verbrückendem N-Atom durchgeführt werden.

Bevorzugt ist das erfindungsgemäße Verfahren ein Festbettverfahren zur kontinuierlichen katalytischen Hydrierung, d.h. es wird in Gegenwart mindestens eines im Festbett fixierten heterogenen Katalysators durchgeführt. Das Festbettverfahren ist weiter bevorzugt ein Verfahren, bei dem die Reaktorkaskade einfach mit den Reaktanden durchströmt wird (Single-Pass-Verfahren).

Heterogene Katalysatoren können entweder Vollkatalysatoren oder geträgerte Katalysatoren sein. Prinzipiell kann bei dem erfindungsgemäßen Verfahren sowohl ein Vollkatalysator als auch ein geträgerter Katalysator eingesetzt werden.

Es kann nur ein Katalysator eingesetzt werden oder ein Gemisch von Katalysatoren. Bevorzugt wird jedoch nur ein Katalysator eingesetzt.

Insbesondere haben sich Katalysatoren umfassend Aktivmetalle ausgewählt aus Nickel, Cobalt, Palladium, Platin, Ruthenium und/oder Rhodium als besonders geeignet erwiesen.

Zur Erhöhung der Aktivität, Selektivität und/oder Standzeit können die Katalysatoren zusätzlich Dotiermetalle enthalten oder Modifizierungsmittel enthalten oder mit diesen behandelt worden sein. Bevorzugte Dotiermetalle können ausgewählt werden aus der Gruppe bestehend aus Mo, Fe, Ag, Cr, V, Ga, In, Bi, Ti, Zr, Mn und den seltenen Erden. Bevorzugte Modifizierungsmittel sind solche, mit denen die Säure-Base-Eigenschaften der Katalysatoren beeinflusst werden können, insbesondere Alkalimetalle, Erdalkalimetalle, Phosphorsäure und Schwefelsäure sowie deren Verbindungen bzw. Salze.

Die Katalysatoren können bevorzugt in Form von Pulvern oder Formkörpern, wie z. B. Extrudaten oder gepressten Pulvern, eingesetzt werden. Es können Vollkontakte, Raney-Typ-Katalysatoren oder Trägerkatalysatoren zur Anwendung kommen.

Bevorzugt wird ein geträgerter Katalysator eingesetzt.

Bevorzugte Trägermaterialien für Trägerkatalysatoren sind Aktivkohle und anorganische Oxide, insbesondere Al₂O₃, SiO₂, TiOz, ZrOz, ZnO und MgO, sowie weiterhin Bentonite, Alumosilikate, Kaoline, Tone, Kieselgure und Lithiumaluminate. Das Aktivmetall kann in einer dem Fachmann bekannten Weise auf das Trägermaterial aufgebracht werden, z. B. durch Imprägnierung, Aufsprühen oder Fällung. Je nach Art der Katalysatorherstellung sind weitere, dem Fachmann bekannte Präparationsschritte notwendig, wie z. B. Trocknung, Calcinierung, Formgebung und Aktivierung. Zur Formgebung können optional weitere Hilfsstoffe wie z. B. Graphit oder Magnesiumstearat zugesetzt werden.

Bevorzugt eingesetzt werden Trägerkatalysatoren mit Ruthenium, Rhodium oder Rh/Ru-Kombinationen als wesentlichen Aktivmetallen. Bevorzugte Trägermaterialien sind solche auf Basis von Al₂O₃ und SiO₂.

Bevorzugt werden solche Katalysatoren eingesetzt, von denen bekannt ist, dass mit ihnen ein Methylenbis(cyclohexylamin) mit einem trans/trans-Anteil des 4,4'-lsomeren zwischen 10 und 30 Gew.-%, insbesondere zwischen 15 und 25 Gew.-% hergestellt werden kann. Solche Katalysatoren sind beispielsweise in den Dokumenten EP 1 366 812 A1, EP 0 066 211 A1, DE 100 54 347 A1, EP 0 392 435 A1, EP 0 630 882 A1, EP 0 639 403 A2 und US 5,545,756 A beschrieben.

Ganz besonders bevorzugt wird die Hydrierung in Gegenwart eines geträgerten Katalysators durchgeführt, der Aktivmetall in einer Menge von 0,01 bis 20 Gew.-%, bezogen auf den geträgerten Katalysator, auf einen Träger aufgebracht enthält, und dessen Aktivmetall Ruthenium alleine oder Ruthenium und mindestens ein Metall der I., VII. oder VIII. Nebengruppe des Periodensystems ist. Mit diesem Katalysator können besonders niedrige trans/trans-Gehalte von 4,4'-Diamino-dicyclohexylmethan erzielt werden.

### a) Reaktorkaskade

Das erfindungsgemäße Verfahren wird in einer Reaktorkaskade durchgeführt, die aus mehreren Reaktionsräumen besteht. Die Reaktorkaskade ist dadurch gekennzeichnet, dass in ihr 50 - 100 mol-%, bevorzugt 50 - 95 mol-%, weiter bevorzugt 70 - 90 mol-% des zur Hydrierung von MDA erforderlichen Wasserstoffs umgesetzt wird. Weiterhin weist sie *n* seriell verschaltete, jeweils mit demselben Katalysator befüllte, unabhängig voneinander befüll- und entleerbare Reaktionsräume auf. Die Zahl n ist dabei eine ganze Zahl größer oder gleich 2, die angibt, wie viele unabhängig voneinander befüll- und entleerbare Reaktionsräume existieren. Bevorzugt beträgt n einen Wert zwischen 2 und 10. Weiter bevorzugt ist *n* eine Zahl ausgewählt aus dem Bereich von 2 bis 6, noch weiter bevorzugt ist *n* = 2, 3 oder 4. Ganz besonders bevorzugt ist *n* = 2 oder 3.

Die einzelnen Reaktionsräume werden in der Reihenfolge ihrer Verschaltung jeweils als *Rᵢ* mit 1 ≤ i ≤ n bezeichnet. Der Begriff "in der Reihenfolge ihrer Verschaltung" ist dabei als synonym zu "in der Reihenfolge des Durchflusses von Edukt (MDA)" anzusehen. Dies bedeutet, der erste Reaktionsraum, durch den eingesetztes MDA zur Reaktion gegeben wird, wird mit R₁ bezeichnet, der zweite wird mit R₂ bezeichnet, und so fort. Der letzte Reaktionsraum wird mit Rₙ bezeichnet. In dem Fall, dass drei Reaktionsräume vorliegen, ist der letzte Reaktionsraum der Reaktorkaskade, den das eingesetzte MDA betritt, somit R₃.

Unter einem Reaktionsraum ist vorliegend eine mit Katalysator befüllte, räumliche Einheit zu verstehen, die von anderen Reaktionsräumen der Reaktorkaskade, in denen in Bezug auf den Reaktionsfortschritt davor bzw. danach MDA hydriert wird, durch katalysatorfreie Anlagenbauteile räumlich separiert ist. Bei einem Reaktionsraum kann es sich somit um einen Teilreaktor handeln, der von anderen Teilreaktoren der Reaktorkaskade, in denen in Bezug auf den Reaktionsfortschritt davor bzw. danach hydriert wird, durch katalysatorfreie Anlagenbauteile räumlich separiert ist. Möglich ist aber auch, dass der Reaktionsraum aus mehreren parallel verschalteten Teilreaktoren besteht, in denen in Bezug auf den Reaktionsfortschritt zum gleichen Zeitpunkt MDA hydriert wird. Bevorzugt besteht jeder Reaktionsraum Rᵢ aus x parallel verschalteten Teilreaktoren mit x = 1 bis 250, die voneinander und von den anderen Reaktionsräumen durch katalysatorfreie Anlagenbauteile räumlich separiert sind. Bei den parallel verschalteten Teilreaktoren kann es sich zum Beispiel um die x parallel verschalteten Rohre eines Rohrbündelreaktors handeln. Weiter bevorzugt beträgt die Zahl x der parallel verschalteten Teilreaktoren x = 2 bis 250, bevorzugt x = 50 bis 250, weiter bevorzugt x = 150 bis 250.

Bevorzugt erfolgt die katalytische Hydrierung von MDA in der Reaktorkaskade im Wesentlichen isotherm, d.h. unter Entziehung von Reaktionsenthalpie mittels eines externen Kühlkreislaufs bei im Wesentlichen gleicher Temperatur.

Bevorzugt wird die Reaktorkaskade nur einmal mit den Reaktanden durchströmt, d. h. die Reaktorkaskade wird im Single-Pass-Modus betrieben.

Ebenfalls bevorzugt handelt es sich bei der Reaktorkaskade um eine Rieselbettkaskade.

### b) Herausnehmen von R₁

Während der Hydrierung von MDA verändert sich die Aktivität von Katalysator in den einzelnen Reaktionsräumen der Reaktorkaskade und nimmt im Laufe der Zeit ab. Es wurde dabei festgestellt, dass die Aktivität eines frisch eingefüllten Katalysators am stärksten im ersten Reaktionsraum und am wenigsten im letzten Reaktionsraum abnimmt. Aus diesem Grund wird R₁ zeitweilig aus der Verschaltung herausgenommen, mit dem Ziel, den in ihm befindlichen Katalysator auszutauschen und/oder zu regenerieren, sobald der in R₁ befindliche Katalysator im Verlauf der Reaktion zu einem unerwünschten Ausmaß deaktiviert wurde.

Kern der vorliegenden Erfindung ist somit, dass es genügt, den Katalysator in R₁ auszutauschen und/oder zu regenerieren, sobald der in R₁ befindliche Katalysator im Verlauf der Reaktion zu einem unerwünschten Ausmaß deaktiviert wurde.

Bevorzugt wird das Erreichen des unerwünschten Ausmaßes der Deaktivierung dadurch bestimmt, dass die Aktivität des Katalysators in R₁ während des Betriebs überwacht und mit seiner anfänglichen Aktivität verglichen wird. Noch weiter bevorzugt erfolgt dies dadurch, dass die normalisierte Aktivität α des in R₁ befindlichen Katalysators bestimmt wird und R₁ zeitweilig aus der Verschaltung herausgenommen wird, sobald die normalisierte Aktivität α des in R₁ befindlichen Katalysators unter einen bestimmten Wert fällt.

Bevorzugt ist somit ein Verfahren zur kontinuierlichen, heterogenen katalytischen Hydrierung von MDA, bei dem
a) das Verfahren in einer Reaktorkaskade durchgeführt wird, die n seriell verschaltete, jeweils mit Katalysator befüllte, unabhängig voneinander befüll- und entleerbare Reaktionsräume aufweist, die in der Reihenfolge ihrer Verschaltung jeweils als *Rᵢ* mit 1 ≤ i ≤ n bezeichnet werden,
b) die normalisierte Aktivität α des in R₁ befindlichen Katalysators bestimmt wird und R₁ zeitweilig aus der Verschaltung herausgenommen wird, sobald die normalisierte Aktivität α des in R₁ befindlichen Katalysators unter einen bestimmten Wert fällt,
   ∘ so dass eine neu verschaltete Reaktorkaskade erhalten wird, die i' Reaktionsräume aufweist, die in der Reihenfolge ihrer Verschaltung jeweils als *R_{i'}* mit 1 ≤ i' ≤ (n - 1) bezeichnet werden,
   ∘ wobei jeder Reaktionsraum Rᵢ mit 2 ≤ i ≤ n zu einem Reaktionsraum R_{i'} mit 1 ≤ i' ≤ (n - 1) wird,
c) der Katalysator in R₁ getauscht und/oder regeneriert und
d) nachfolgend R₁ als Reaktionsraum R_{i'} mit i' = n verschaltet wird.

Bevorzugt wird während des Betriebs die Aktivität des Katalysators in R₁ überwacht.

Dies kann z. B. über ein kontinuierliches oder punktuelles Online-Monitoring des Katalysators oder über eine Entnahme von Katalysatorproben mit anschließender externer Untersuchung in einem Referenzversuch im Labormaßstab zu verschiedenen Zeiten tₓ erfolgen. Die normalisierte Aktivität α des Katalysators kann als Quotient des Umsatzes zum Zeitpunkt t = tₓ (Dividend) und des Umsatzes zum Zeitpunkt t = 0 (Divisor) bestimmt werden, wobei die Umsatzbestimmung bei einer Reaktionstemperatur von 100 °C, einem Wasserstoffdruck von 80 bar und einer inversen Masse-bezogenen Verweilzeit von 0,330 kg (MDA) / (kg (Katalysator)_{*}h) an MDA pro Gesamtmasse an Katalysator aller Reaktionsräumen der Reaktorkaskade durchgeführt wird.

Die bevorzugte Position zur Bestimmung des Umsatzes zur Überwachung der Aktivität des Katalysators in R₁ erfolgt dabei immer an derselben Stelle. Weiter bevorzugt wird die Aktivität des Katalysators unmittelbar am Ausgang des Reaktionsraumes R₁ überwacht.

Der tatsächliche Umsatz bezogen auf den Wasserstoffverbrauch wird berechnet, in dem die Flächenprozente an Methylenbis(cyclohexylamin) im Gaschromatogramm mit dem Faktor 1 gewertet, zu diesen die 0,5-fachen Flächenprozente der Methylen(aminocyclohexyl)anilin-Komponenten addiert und die erhaltene Summe durch die Summe der Flächenprozente aller MDA-, Methylen-(aminocyclohexyl)anilin- und Methylenbis(cyclohexylamin)-Verbindungen geteilt wird.

Sobald der in R₁ befindliche Katalysator im Verlauf der Reaktion zu einem unerwünschten Ausmaß deaktiviert wurde, wird die Verschaltung der einzelnen Reaktionsräume geändert. Bevorzugt wird die Verschaltung geändert, sobald die normalisierte Aktivität α des in R₁ befindlichen Katalysators unter einen bestimmten Wert fällt. Nach der Änderung der Verschaltung wird der Katalysator in R₁ getauscht und/oder regeneriert.

Für die Ausführbarkeit der vorliegenden Erfindung und die Erzielung der erfindungsgemäßen Vorteile ist dabei eine Quantifizierung des Ausmaßes der Deaktivierung oder der tatsächliche Zahlenwert der normalisierten Aktivität α weitestgehend unerheblich. Sobald der Umsatz an MDA in einen unerwünschten Bereich absinkt, ist allein daraus ersichtlich, dass der Katalysator deaktiviert wurde bzw. seine normalisierte Aktivität α abgenommen hat und eine Aktion erforderlich ist.

Bevorzugt wird R₁ jedoch dann aus der Verschaltung genommen, wenn die normalisierte Aktivität α des in R₁ befindlichen Katalysators kleiner als 0,7 ist, besonders bevorzugt kleiner 0,5, noch weiter bevorzugt kleiner 0,3.

Sobald der in R₁ befindliche Katalysator im Verlauf der Reaktion zu einem unerwünschten Ausmaß deaktiviert wurde, insbesondere die normalisierte Aktivität α des in R₁ befindlichen Katalysators unter einen bestimmten, nicht gewünschten Wert gefallen ist, wird die Verschaltung der einzelnen Reaktionsräume so verändert, dass R₁ zeitweilig aus der Verschaltung genommen wird. Dies erfolgt bevorzugt durch ein Umleiten der Edukt- und Produktströme. Hierzu werden die zulaufenden Ströme an nicht umgesetzten MDA und Wasserstoff direkt auf Reaktor R₂ geleitet. Die zulaufenden und ablaufenden Leitungen zu R₁ werden hingegen geschlossen.

Dadurch, dass R₁ aus der Verschaltung von n Reaktionsräumen herausgenommen wird, wird eine neu verschaltete Reaktorkaskade erhalten, der einen Reaktionsraum weniger, d. h. (n - 1) Reaktionsräume aufweist. Diese können anhand der nun vorliegenden geänderten Verschaltung neu bezeichnet werden: Dadurch, dass R₁ aus der Verschaltung des n Reaktionsräume aufweisenden Reaktorkaskade herausgenommen wird, entsteht aus den verbleibenden Reaktionsräumen Rᵢ mit 2 ≤ i ≤ n eine Zahl von (n-1) Reaktionsräumen, die in der Reihenfolge ihrer Verschaltung als R_{i'} mit 1 ≤ i' ≤ (n - 1) bezeichnet werden.

### c) Tausch/Regenerierung des Katalysators

Der in R₁ befindliche Katalysator wird getauscht und/oder regeneriert, d.h. der Katalysator wird partiell oder vollständig (bevorzugt vollständig) getauscht oder regeneriert. Denkbar ist auch, dass ein Teil des Katalysators partiell oder vollständig (bevorzugt vollständig) regeneriert und ein anderer Teil des Katalysators partiell oder vollständig (bevorzugt vollständig) getauscht wird.

Bevorzugt geschieht dies durch Entspannung und Restentleerung des Reaktors in einen Slopbehälter. Anschließend kann ggf. noch vorhandenes Lösemittel mit Wasser aus dem Katalysator gewaschen werden und der Katalysator im Stickstoffstrom bei erhöhter Temperatur getrocknet werden. Der Katalysator kann im Anschluss ausgebaut und durch einen frischen Katalysator ersetzt werden.

Alternativ kann der Katalysator auch regeneriert werden. Bevorzugte Regenerationsverfahren sind zum Beispiel in CN 117654548 A, CN 110204447 B und CN 113893866 B beschrieben.

Nach Tausch und/oder Regenerierung des ursprünglich in R₁ befindlichen Katalysators wird dieser Reaktionsraum nachfolgend als Reaktionsraum R_{i'} mit i' = n verschaltet. Dies heißt, dass der Reaktionsraum als letzter Reaktionsraum der Reaktorkaskade verschaltet wird. Nachdem der früher als R₁ bezeichnete Reaktionsraum als neuer Reaktionsraum Rₙ verschaltet wurde, weist die Reaktorkaskade also wieder n seriell verschaltete, jeweils mit Katalysator befüllte, unabhängig voneinander befüll- und entleerbare Reaktionsräume auf. Selbstverständlich kann mit dieser neu verschalteten Reaktorkaskade mit n Reaktionsräumen das erfindungsgemäße Verfahren erneut durchgeführt werden, sobald der nun in R₁ befindliche Katalysator nicht mehr ausreichend aktiv ist.

Die vorliegende Erfindung beruht auf einem Reaktorkonzept, bei dem der Reaktor aus zwei oder mehr Reaktionsräumen besteht, die wechselseitig seriell angeordnet sind. In einem solchen Aufbau können die einzelnen Reaktionsräume ausgetauscht werden, ohne dass dabei der noch aktive Anteil des Katalysators aufbereitet oder ausgetauscht wird.

Während des Austauschs bzw. während der Regenerierung des Katalysators kann die Hydrierung prinzipiell angehalten oder fortgeführt werden. Bevorzugt wird die Hydrierung während des Tauschs oder der Regenerierung weiter betrieben, da so die Raum-Zeit-Ausbeute der Anlage besser ist. Weiter bevorzugt wird die Hydrierung mit reduzierter Last weiterbetrieben, da so besonders gute Ergebnisse erzielt werden können.

In einer bevorzugten Ausführungsform des Verfahrens ist n = 2, d. h. der Reaktorkaskade besteht aus zwei Reaktionsräumen R₁ und R₂, die unabhängig voneinander befüll- und entleerbar sind und die in der Reihenfolge ihrer Verschaltung jeweils als R₁ und R₂ bezeichnet werden. R₁ wird zeitweilig aus der Verschaltung herausgenommen, sobald der in R₁ befindliche Katalysator im Verlauf der Reaktion zu einem unerwünschten Ausmaß deaktiviert wurde. Die Reaktion wird somit nur in R₂ durchgeführt und R₂ von nun an als Reaktionsraum R_{1'} bezeichnet. Der Katalysator in R₁ wird getauscht und/oder regeneriert. Nachfolgend wird R₁ als Reaktionsraum R_{2'} verschaltet.

Weiter bevorzugt wird das Erreichen des unerwünschten Ausmaßes der Deaktivierung dadurch bestimmt, dass die Aktivität des Katalysators in R₁ während des Betriebs überwacht und mit seiner anfänglichen Aktivität verglichen wird. Noch weiter bevorzugt wird die normalisierte Aktivität α des in R₁ befindlichen Katalysators bestimmt und R₁ zeitweilig aus der Verschaltung herausgenommen, sobald die normalisierte Aktivität α des in R₁ befindlichen Katalysators unter einen bestimmten Wert fällt.

Die erfindungsgemäße kontinuierliche Hydrierung kann ausschließlich in der genannten Reaktorkaskade durchgeführt werden. Bevorzugt ist jedoch, dass nach der Umsetzung in der Reaktorkaskade eine Umsetzung in einem Finisher (der ggf. mehrere Teil-Finisher aufweist) erfolgt. In diesem wird, bevorzugt in Gegenwart mindestens eines anderen Katalysators, der Umsatz der Hydrierung von MDA weiter erhöht. Dieser Finisher (Nachreaktor) ist nicht Bestandteil der Reaktorkaskade.

Bevorzugt wird pro Tonne Katalysator in der Reaktorkaskade in den Finisher ein Stoffstrom von 0,15 - 25 t/h, weiter bevorzugt 0,75 - 20 t/h, noch weiter bevorzugt 1 - 15 t/h eingeleitet.

Besonders gute Ergebnisse können erzielt werden, wenn die Umsetzung in der Reaktorkaskade im Wesentlich isotherm (d.h. unter Entziehung von Reaktionsenthalpie mittels eines externen Kühlkreislaufs bei im Wesentlichen konstanter Temperatur) und im Finisher im Wesentlichen adiabatisch (d.h. ohne Kühlung mit wenig Wärmeverlust) geführt wird.

## Patentansprüche

1. Verfahren zur kontinuierlichen, heterogenen katalytischen Hydrierung von MDA,
**dadurch gekennzeichnet, dass**
a) das Verfahren in einer Reaktorkaskade durchgeführt wird, die n seriell verschaltete, jeweils mit Katalysator befüllte, unabhängig voneinander befüll- und entleerbare Reaktionsräume aufweist, die in der Reihenfolge ihrer Verschaltung jeweils als *Rᵢ* mit 1 ≤ i ≤ n bezeichnet werden,
b) und R₁ zeitweilig aus der Verschaltung herausgenommen wird, sobald der in R₁ befindliche Katalysator im Verlauf der Reaktion zu einem unerwünschten Ausmaß deaktiviert wurde,
∘ so dass eine neu verschaltete Reaktorkaskade erhalten wird, die i' Reaktionsräume aufweist, die in der Reihenfolge ihrer Verschaltung jeweils als R;- mit 1 ≤ i' ≤ (n - 1) bezeichnet werden,
∘ wobei jeder Reaktionsraum Rᵢ mit 2 ≤ i ≤ n zu einem Reaktionsraum R_{i'} mit 1 ≤ i' ≤ (n - 1) wird,
c) der Katalysator in R₁ getauscht und/oder regeneriert und
d) nachfolgend R₁ als Reaktionsraum R_{i'} mit i' = n verschaltet wird.

2. Verfahren nach Anspruch 1,
**dadurch gekennzeichnet, dass**
das eingesetzte MDA mindestens 70 Gew.-% 4,4'-Diaminodiphenylmethan und 0,01 bis 2 Gew.-% N-Methylverbindungen, jeweils bezogen auf die Gesamtmasse an Verbindungen mit aromatischen Ringen, aufweist.

3. Verfahren nach Anspruch 1 oder 2,
**dadurch gekennzeichnet, dass**
das Verfahren ein Festbettverfahren zur kontinuierlichen katalytischen Hydrierung ist.

4. Verfahren nach einem der vorhergehenden Ansprüche
**dadurch gekennzeichnet, dass**
der Katalysator ein geträgerter Katalysator ist.

5. Verfahren nach Anspruch 4,
**dadurch gekennzeichnet, dass**
der Katalysator
- Aktivmetall in einer Menge von 0,01 bis 20 Gew.-%, bezogen auf den geträgerten Katalysator, auf einen Träger aufgebracht enthält, und
- dessen Aktivmetall Ruthenium alleine oder Ruthenium und mindestens ein Metall der I., VII. oder VIII. Nebengruppe des Periodensystems ist.

6. Verfahren nach einem der vorhergehenden Ansprüche,
**dadurch gekennzeichnet, dass**
jeder Reaktionsraum Rᵢ aus x parallel verschalteten Teilreaktoren mit x = 1 bis 250 besteht, die voneinander und von den anderen Reaktionsräumen durch katalysatorfreie Anlagenbauteile räumlich separiert sind.

7. Verfahren nach einem der vorhergehenden Ansprüche,
**dadurch gekennzeichnet, dass**
die katalytische Hydrierung von MDA in der Reaktorkaskade im Wesentlichen isotherm erfolgt.

8. Verfahren nach einem der vorhergehenden Ansprüche,
**dadurch gekennzeichnet, dass**
das Erreichen des unerwünschten Ausmaßes der Deaktivierung dadurch bestimmt wird, dass die Aktivität des Katalysators in R₁ während des Betriebs überwacht und mit seiner anfänglichen Aktivität verglichen wird.

9. Verfahren nach Anspruch 8,
**dadurch gekennzeichnet, dass**
die normalisierte Aktivität α des in R₁ befindlichen Katalysators bestimmt wird und R₁ zeitweilig aus der Verschaltung herausgenommen wird, sobald die normalisierte Aktivität α des in R₁ befindlichen Katalysators unter einen bestimmten Wert fällt.

10. Verfahren nach Anspruch 9,
**dadurch gekennzeichnet, dass**
die normalisierte Aktivität α des in R₁ befindlichen Katalysators kleiner als 0,7 ist.

11. Verfahren nach einem der vorhergehenden Ansprüche,
**dadurch gekennzeichnet, dass**
die Hydrierung während des Tauschs oder der Regenerierung des Katalysators weiterbetrieben wird.

12. Verfahren nach einem der vorhergehenden Ansprüche,
**dadurch gekennzeichnet, dass**
a) das Verfahren in einer Reaktorkaskade durchgeführt wird, die zwei seriell verschaltete, jeweils mit Katalysator befüllte, unabhängig voneinander befüll- und entleerbare Reaktionsräume aufweist, die in der Reihenfolge ihrer Verschaltung jeweils als R₁ und R₂ bezeichnet werden,
b) und R₁ zeitweilig aus der Verschaltung herausgenommen wird, sobald der in R₁ befindliche Katalysator im Verlauf der Reaktion zu einem unerwünschten Ausmaß deaktiviert wurde,
o so dass die Reaktion nur in R₂ durchgeführt wird, wobei R₂ von nun an als Reaktionsraum R_{1'} bezeichnet wird,
c) der Katalysator in R₁ getauscht und/oder regeneriert und nachfolgend R₁ als Reaktionsraum R_{2'} verschaltet wird.

13. Verfahren nach einem der vorhergehenden Ansprüche,
**dadurch gekennzeichnet, dass**
nach der Umsetzung in der Reaktorkaskade eine Umsetzung in einem Finisher erfolgt.

14. Verfahren nach Anspruch 13,
**dadurch gekennzeichnet, dass**
die Umsetzung in der Reaktorkaskade im Wesentlichen isotherm und im Finisher im Wesentlichen adiabatisch geführt wird.
